# EUROPEAN PATENT SPECIFICATION

(11) **EP 2 062 881 B1**
(45) Date of publication and mention of the grant of the patent: **23.02.2011**
(21) Application number: 07022670.9
(22) Date of filing: 22.11.2007
(51) Int. Cl.: C07D 295/205, C07D 295/073

(54) **Process for making N-(diphenylmethyl)piperazines**
Verfahren zur Herstellung von N-(Diphenylmethyl)piperazinen
Procédé pour la fabrication de N-(diphénylméthyl)pipérazines

(30) Priority: 21.11.2007 US 989528 P
(43) Date of publication of application: 27.05.2009
(73) Proprietor: Synthon B.V., 6545 CM Nijmegen (NL)
(72) Inventor: Zhu, Jie, 6538 HL Nijmegen (NL)
(74) Representative: Prins, Hendrik Willem

(56) References cited:
- EP-A- 1 236 722
- WO-A-2007/066163
- DE-B- 1 139 113
- GB-A- 2 076 403
- DATABASE CAPLUS [Online] CHEMICAL ABSTRACTS SERVICE, COLUMBUS, OHIO, US; 1972, XP002464434 retrieved from STN accession no. 140617 & A. P. SINEOKOV ET AL.: "Synthesis of N-(2-chloromethyl)- and N-vinyl-2-oxazolidinone" KHIMIYA GETEROTSIKLICHESKIKH SOEDINENII, no. 2, 1970, pages 275-277, MOSCOW; RU
- DATABASE REGISTRY [Online] CHEMICAL ABSTRACTS SERVICE, COLUMBUS, OHIO, US; CAS Registry Number 500877-20-3 28 March 2003 (2003-03-28), XP002464435 retrieved from STN
- DATABASE CAPLUS [Online] CHEMICAL ABSTRACTS SERVICE, COLUMBUS, OHIO, US; 1964, XP002464436 retrieved from STN Database accession no. 60431 & P. BERCOT: "N,N-Bis(.beta.-chloroethyl)carbamoyl chloride; its use for the syntesis of some alkyl N,N-bis(.beta.-chloroethyl)carbamates" COMPTE RENDUS, vol. 258, no. 1, 1964, pages 224-226, PARIS; FR
- DATABASE CAPLUS [Online] CHEMICAL ABSTRACTS SERVICE, COLUMBUS, OHIO, US; 1966, XP002464437 retrieved from STN Database accession no. 489972 & N. SUCIU: "Nitrogen mustards of the urethan type" REVUE ROUMAINE DE CHIMIE, vol. 11, no. 6, 1966, pages 745-749, BUKAREST; RO
- DATABASE CAPLUS [Online] CHEMICAL ABSTRACTS SERVICE, COLUMBUS, OHIO, US; 1959, XP002464438 retrieved from STN Database accession no. 6676 & G. OLAH ET AL.: "Synthesis of organic fluorine compounds. XIII. derivatives of 2-fluoroethylurethane" ACADEMIAE SCIENTIARUM HUNGARICAE. ACTA CHIMICA, no. 7, 1955, pages 443-449, AKADEMIAI KIADO RT, BUDAPEST; HU
- DATABASE CAPLUS [Online] CHEMICAL ABSTRACTS SERVICE, COLUMBUS, OHIO, US; 2003, XP002464439 retrieved from STN Database accession no. 500673 & K.J.P YOON ET AL.: "Synthesis and evaluation of esters and carbamates to identify critical functional groups for esterase-specific metabolism" BIOORGANIC & MEDICINAL CHEMISTRY., vol. 11, no. 15, 2003, pages 3237-3244, ELSEVIER SCIENCE LTD.; GB

## Description

Cetirizine, chemically 2-[4-[(4-chlorophenyl)-phenyl-methyl] piperazin-1-yl]ethoxy]acetic acid, is a useful pharmaceutical active ingredient. It is an antihistamine whose principal effects are mediated via selective inhibition of H, receptors. This anti-allergy drug is marketed by the company UCB (which is also the originator of the drug) and/or Pfizer under the brand name Zyrtec®, as a dihydrochloride salt (often referred to as "cetirizine hydrochloride") as shown below. 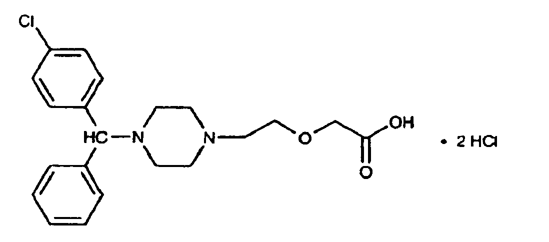 The drug is indicated for the relief of symptoms associated with seasonal allergic rhinitis or perennial allergic rhinitis, as well as for the treatment of the uncomplicated skin manifestations of chronic idiopathic urticaria in adults and children 6 months of age and older.

Cetirizine has one asymmetric carbon, therefore it may be resolved into enantiomers. The pharmaceutically active enantiomer in the racemic cetirizine is the levocetirizine, which is the (R) enantiomer of cetirizine. A medicament comprising levocetirizine was launched in the first quarter of 2001 in Germany followed by a pan-European launch. Levocetirizine is also marketed as the dihydrochloride salt, under the brand name Xyzaal®.

Cetirizine was disclosed in US 4,525,358 (EP 58146). Levocetirizine was specifically disclosed in GB2225321. The method of use of levocetirizine has been disclosed in US 5,698,558 (EP 663828).

Conventionally, levocetirizine may be obtained by resolution of the cetirizine enantiomers as generally suggested, e.g., in WO 94/06429. However, the effectiveness of such process is apparently not high and therefore it is preferred to make levocetirizine from an enantiopure intermediate.

One such useful intermediate is the compound of formula (4)

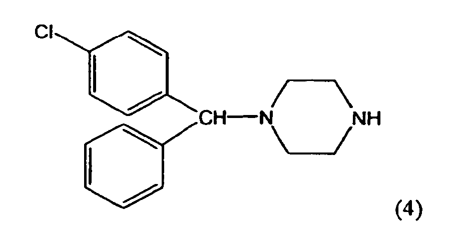

The presence of a quarternary carbon in the formula (4) indicates that the compound may be obtained as a racemate or as a single enantiomer, particularly as the (R) enantiomer. This intermediate may be converted to cetirizine or related analogues, particularly to racemic cetirizine or levocetirizine, by various known processes, e.g., by processes reviewed in US 4,525,358. Resolution of the intermediate (4) into enantiomers by L-tartaric acid as well as the process for making levocetirizine from the corresponding enantiomer of (4) was disclosed in GB2225321. However, the yield and effectiveness of the resolution is insufficient, as shown in US 5,478,941.

The useful starting material for making the compound (4) is the well known and commercially available compound of formula (1),

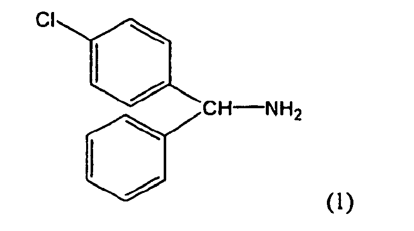

Similarly as the above compound (4), the compound (1) may be obtained as a racemate or as a single enantiomer, particularly as the (R) enantiomer. It is known that the racemic compound (1) can be easily and effectively resolved into enantiomers by a fractional crystallization, preferably by the crystallization of salts with L- tartaric acid. (see US 5,478,941). This makes the compound (1) an important intermediate, particularly in the synthesis of an enantiomerically pure (4).

In a known process for making compound (4) disclosed in EP 617028 (US 5,478,941), the racemic compound of formula (1) and/or its (R)-enantiomer is subjected to a condensation with the N-sulfonated bis-chloroethylamine compound of formula (2), 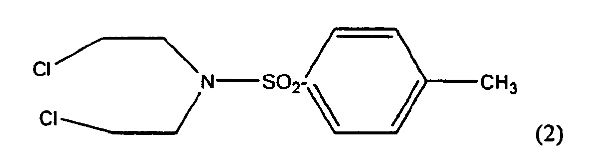 to form the compound of formula (3). 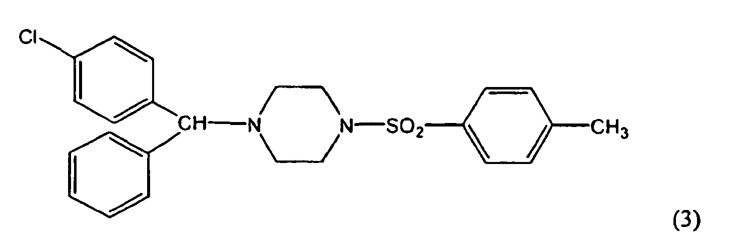 The compound (3) is then deprotected to form the key intermediate of general formula (4). A disadvantage, however, with the use of the compound of formula (2) in the synthesis of the compound of formula (4) is the need to use a strong deprotecting agent. The tosyl-protective group may be effectively removed only by using a solution of hydrogen bromide in acetic acid. This agent is extremely corrosive, irritating and toxic so that special measures must be used in employing this material.

In principle, one could expect that also an unprotected compound of the formula (5a) 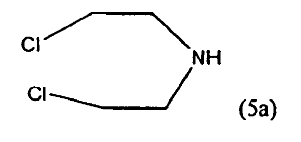 might be used for coupling with the compound (1). This would avoid the deprotection step and form the compound (4) directly. But this option is not satisfactory. First, the compound (5a) is an extremely toxic compound ("mustard gas"), and second the reaction is accompanied with a large amount of side products arising particularly from the self-condensation of the compound (5). Thus, the use of an N- protected bis-haloethylamine is clearly preferable. But other potentially useful N- protected compounds, e.g. a carbonyl, alkyl or a triphenylmethyl protecting group, have been reported as unsatisfactory. US 5,478,941 and EP 955295 teach that the above mentioned N-tosyl compound of formula (2) is the only useful compound for the coupling reaction with (1). The protected analogues (a carbonyl, alkyl, or trityl protecting group) caused important racemization of the compound (1) during the coupling reaction and/or the formation of undesired by-products.

WO 2007/066163 (EP1963296) discloses a process for the preparation of optically active carbamates, which are intermediates for the preparation of cetirizine. These intermediates are formed by a reaction of 1-(4-chlorophenyl)-methylphenyl-amin with bis-(2-chloroethyl)amine protected with the easily removable leaving group 2,2,2-trichloroethoxy. It is reported that due to the drastic cyclisation reaction conditions this leaving group is almost totally removed.

Opalka C.J. et al. (Synthesis 1995 (7), p. 766-768) reports that the coupling reaction failed if the amides of formula 6, wherein R represents a carbon-terminated substituent, were used. 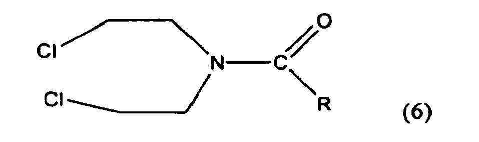 Thus other protecting groups have proven to be unsuitable so far. It would be desirable to have an alternative process for making the compound of general formula (A), particularly for making the racemate or the R-enantiomer thereof.

### Summary of the Invention

The present invention relates to the discovery of a suitable protecting group and a convenient process for making cetirizine including levocetirizine. Accordingly, the invention relates to a process, which comprises:
a) reacting a compound of formula (1) with a compound of formula (7) in the presence of a base, 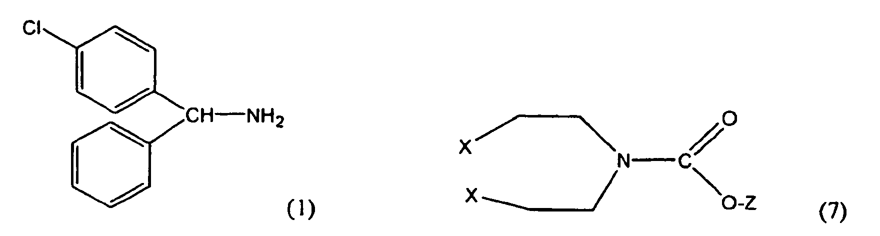 to form a compound of formula (8): 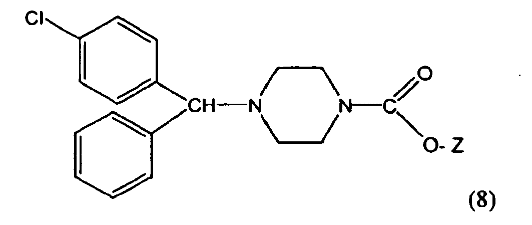 wherein X is a leaving group reactive with an amine, preferably a halo group such as chloro or bromo group; or a sulphonyl group such as mesyloxy, besyloxy or tosyloxy group; and preferably X is a chloro group;
b) deprotecting said compound of formula (8), preferably by an alkaline hydrolysis, to form a compound of formula (4) 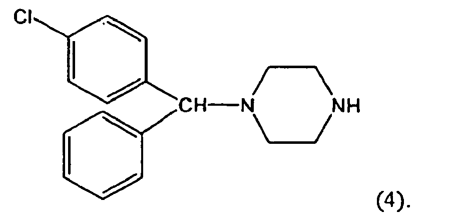
The compound of formula (4) can be converted to a cetirizine compound. The compounds of formula (1), (8), and (4) can be racemic or substantially a single enantiomer, typically the (R) enantiomer. Each of the above formulas includes salts of the compounds. Furthermore, these compounds may be used in the preparation of cetirizide and salts thereof.

Another aspect of the invention relates to the compounds of formulas (7) and (8). Typically X is chloro and Z is phenyl. The compounds can be racemic or substantially the (R) enantiomer.

A further aspect of the invention relates to a crystalline oxalic acid addition salt of a compound of formula (4). The oxalate allows for isolation of the compound of formula (4) and can be stored.

### Detailed Description of the Invention.

The present invention deals with an alternate process for making the compound of formula (4), which is the key intermediate in the synthesis of pharmaceutically useful compounds including cetirizine compounds. The use of the ester protecting group allows for less stringent deprotection conditions without causing racemization. Thus, the compound of formula (1) can be converted into the compound (4) by a more convenient process without the use of the corrosive, toxic agent of HBr in acetic acid.

As used herein, all chemical formulas having a chiral carbon, e.g., (1), (4), (7), and (8), include both mixtures of the enantiomers such as a racemate as well as substantially a single enantiomer; i.e., at least 90% optical purity, preferably at least 95% optical purity, and including at least 98% and at least 99% optical purity. Similarly, all chemical formulae, e.g., (1) to (8), include the acid addition salts thereof unless explicitly stated to the contrary. For instance, the starting compound (1) may react as a base, or as an acid additions salt, for instance as the hydrochloride.

In the first step of the process of the present invention, the compound of formula (1), a racemate, a single enantiomer thereof or mixtures of any of the preceded, reacts, generally in a liquid phase, with the compound of the general formula (7) to yield the compound of formula (8). 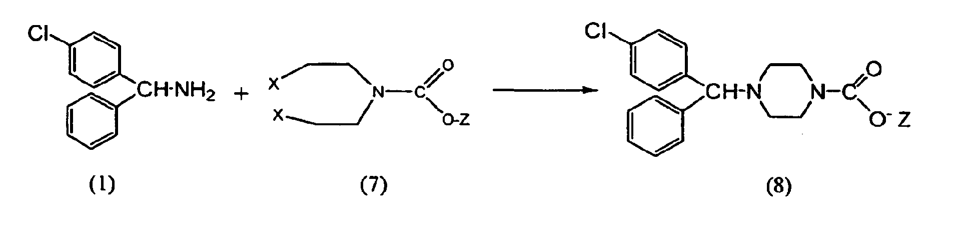 In the formula (7) and (8), Z is C1-C20 straight or branched alkyl group, C7-C20 aralkyl group, C6-C20 aryl /alkylaryl group, each of groups moreover with a possibility to be substituted by one to four halogen, alkoxy, amino, and/or nitro groups. Preferably, the Z is a phenyl group.

The compound (7) also contains two equal leaving groups X that are reactive with the primary amine in the compound (1) to form the piperazine ring. Such groups X may be represented by a halogen group, or a sulphonyl group such as mesyloxy, besyloxy, anisylsulfonyloxy or tosyloxy group; preferably X is a chloro-group.

Thus, the preferred example of the compound of the general formula (7) is bis(chloroethyl)amine phenyl carbamate of formula (7a),

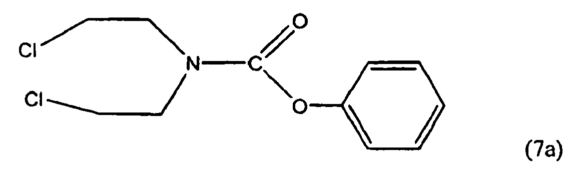

The reaction between compounds (1) and (7) proceeds in the presence of a base, which is preferably an organic base. In a convenient embodiment, a liquid organic base is employed, whereby the liquid organic base serves also as the solvent of the reaction. The preferred liquid organic base is diisopropylethylamine. The reaction preferably proceeds at an enhanced temperature, e.g., at a temperature between 50-150°C, suitably at reflux. The reaction progress may be monitored by a suitable analytical technique, e.g. HPLC. After the reaction, the reaction mixture containing the product (8) may be used for the next step (advantageously, after removal of amine salts formed and/or after removal of at least part of the solvent) or is elaborated to isolate the reaction product (8). In a suitable way of isolation, the reaction mixture is partitioned between an aqueous and organic phase (whereby the organic solvent may be conveniently a hydrocarbon or a chlorinated hydrocarbon) and the product is isolated from the organic phase. The crude product may be purified, if necessary, or may be used in the next step in the crude state. The compound (8) may also be isolated as an acid addition salt.

Dependent on the conformation of the compound (1), the product of formula (8) is a racemate, a single enantiomer thereof or mixtures of any of the preceded. It is an important advantage that the reaction between (1) and (7) proceeds substantially without racemization, so that a substantially single enantiomer of (1) yields the corresponding substantial single enantiomer of (8).

If the preferred compound (7a) is used, the reaction product is the compound of formula (8a), a racemate or a single enantiomer, particularly substantially the (R)- enantiomer, or a mixture of the preceded.

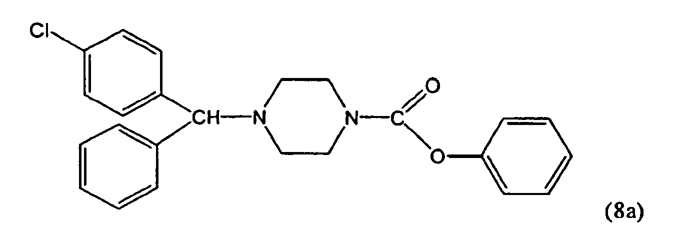

In the second step, the compound (8) is subjected to a deprotection step. The N-protective alkoxycarbonyl/aryloxycarbonyl moiety is generally sensitive to alkaline hydrolysis and may be removed by an aqueous alkali, thus avoiding the use of the toxic and irritant HBr/acetic acid agent. The "aqueous alkali" comprises an aqueous solution or suspension of lithium, sodium, potassium or calcium hydroxide or carbonate. The reaction may proceed in the presence of an inert co-solvent, such as in an alcoholic solvent (methanolic, ethanolic, isopropanolic solvent), or the aqueous alkali serves itself as the solvent. The reaction product (4) is then advantageously extracted by a water-insoluble organic solvent and isolated from the organic phase.

Both steps may be also performed in a "one-pot arrangement" as indicated above, i.e., the reaction mixture from the first step is subjected, without an isolation of the intermediate product, to the alkaline hydrolysis by the aqueous alkali.

In an advantageous mode, the formed compound of formula (4), a racemate, a single enantiomer thereof or mixtures of any of the preceded, is isolated from the reaction mixture, and/or purified. For instance, it may be isolated by converting it into an acid addition salt with an organic or inorganic acid that is isolatable as a solid, preferably crystalline, product. An advantageous salt in this respect is the oxalate salt as it may be isolated as a stable crystalline material. The oxalate salt of the compound (4) is a suitable form that allows storage of the compound (4), particularly the racemate or the (R)- enantiomer thereof, for an enhanced period of time. The compound (4) may be however isolated also as a free base, which is preferably a solid product, for instance by a suitable extraction process. In an example, the reaction mixture is partitioned between an organic layer and acidified aqueous layer (in which the product concentrates), the aqueous layer is neutralized, the free base of (4) is extracted by an organic solvent and isolated from this solvent.

The starting (4-chlorophenyl)phenylmethylamine of formula (1) is a known, commercially available compound. It may be used as a racemate or as a single enantiomer, either as a free base or as an acid addition salt, e.g., as the hydrochloride. Both the levorotatory (-)(4-chlorophenyl)phenylmethylamine and the dextrorotatory (+)(4-chlorophenyl)phenylmethylamine may be prepared by the resolution of the racemic compound by (+) or (-) tartaric acid, resp., according to the method described by Clemo et al, J.Chem.Soc. (1939), p.1958-1960.

The compound of formula (7) may be obtained, for instance, by the condensation of the compound (5) 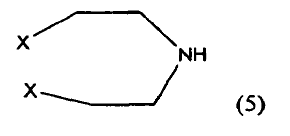 and/or an acid addition salt thereof, with a chloroformate compound of formula (9) 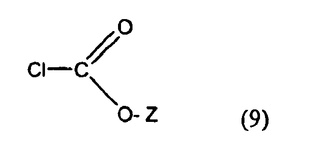 wherein X and Z have the above meaning. The preferred compound bis(chloroethyl)amine phenyl carbamate of the formula (7a) is thus obtained by the reaction of the bis(chloroethylamine) of formula (5a) with a phenylchloroformate of formula (9a).

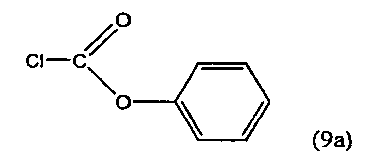

The reaction is advantageously performed in an inert solvent, e.g., in a hydrocarbon solvent or a halogenated hydrocarbon solvent, preferably in the presence of a base. The reaction generally proceeds at the ambient or close to ambient temperature. The product is isolated from the reaction mixture by conventional means, e.g., by an extraction into a suitable organic solvent and removal the solvent.

Alternatively, the compounds of formula (7) may be obtained from bis (hydroxyethyl) amine and a haloformate (9) according to the scheme 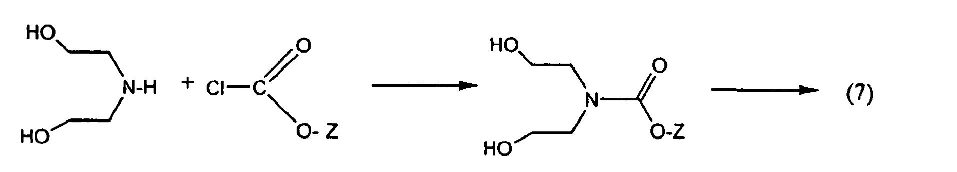 under general conditions known in the art.

The compound of formula (4), as well as acid addition salts thereof, prepared by the above process, may be converted into a cetirizine compound by known means as described in the above cited patents. As used herein a "cetirizine compound" embraces the cetirizine racemate and its salts as well as substantially one enantiomer (levocetirizine) and salts thereof.

In light of the prior art disclosure, which teaches away of using carbonyl-based N-protecting groups in making the compound of formula (4), the finding of a suitable group of successfully useful carbonyl- comprising compounds for the same purpose is surprising. In particular, the compounds (4) may be obtained by the present process in a high degree of chemical and/or enantiomeric purity, without a danger of racemization and under conditions that are easily and reliably obtainable in an industrial process and avoid using irritating and toxic HBr/acetic acid solution. Consequently, the whole manufacturing process for making pharmaceutically useful compounds cetirizine and/or levocetirizine, if comprising the process of the present invention, becomes more economical and more technically convenient.

The invention is illustrated by the following examples.

### Example 1 - Bis(chloroethyl)amine phenyl carbamate - compound of formula (7a)

To a suspended bis(chloroethyl)amine hydrochloride salt (17.85, 0.1 mole) in 200 ml dichloromethane, a 1M NaOH solution (120 ml) was added and stirred at room temperature ("rt") for 1 hour. Separated dichlormethane layer was dried and concentrated *in vacuo* to give an oily bis(chloroethyl)amine free base.

Above crude oil was dissolved in 50 ml dried dichloromethane. With cooling (ice water) and stirring, phenyl chloroformate (12.55 ml, 0.1 mole) was added dropwise. The addition was completed within 20 min followed by addition of triethylamine (22.0 g, 0.22 mole). The mixture was further stirred at room temperature for 2 hours. Water (100 ml) was added, and the mixture was stirred for 20 min. Separated water layer was extracted with dichloromethane (50 ml). Combined dichloromethane layer was washed with water (10 ml), dried and concentrated *in vacuo* to give an oily product (26.5 g, ∼100% yield).

### Example 2:

### Step 1 - compound (8a)

A mixture containing (4-chlorophenyl)phenyl methylamine (2.17 g, 10 mmol), bis(chloroethyl)amine phenyl carbamate [7a] (2.75 g, 10.5 mmol) and diisopropyl ethylamine (5 ml) was stirred (N₂ gas was pre-blown through for 1 hour before heating up), with smooth refluxing (130°C oil bath), for 5 hours.

After cooling down to room temperature, 50 ml dichloromethane and 25 ml water was added, and the mixture was stirred for 20 min. Separated dichloromethane layer was concentrated to give an oil (∼4.6 g).

### Step 2 - compound (4)

Above oil was dissolved in 20 ml 2-propanol, followed by addition of 5 ml NaOH solution (prepared from 10 g NaOH and 20 ml water). The mixture was stirred, with smooth refluxing (90°C oil bath), for 2 hours.

The mixture was concentrated *in vacuo.* 50 ml isopropyl ether and 25 ml water was added, and the mixture was stirred for 30 min. Separated ether layer was washed with water, brine, dried and concentrated to give a crude oily product (2.55g).

### Step 3 - compound (4) oxalate

A solution of above crude product in 25 ml ethyl acetate was stirred at room temperature. Oxalic acid (0.9 g, 10 mmol) was added, the mixture was stirred at rt for 2 hours and further at ∼5°C overnight. Solid was collected by filtration and washed once with ethyl acetate (5 ml). 2.3 g oxalate solid was obtained after drying at 40°C overnight *in vacuo.*

### Example 3

### Compound (4) oxalate

A mixture containing (4-chlorophenyl)phenyl methylamine (2.17 g, 10 mmol), bis(chloroethyl)amine phenyl carbamate (2.75 g, 10.5 mmol) and diisopropyl ethylamine (4 ml) was stirred at 90°C for 1.5 hours (N₂ gas was pre-blowed through for 1 hour before heating up), and further with smooth refluxing (130°C oil bath), for 4 hours.

After cooling down to room temperature, 20 ml 2-propanol was added followed by addition of 3 ml NaOH solution (prepared from 10 g NaOH and 10 ml water). The mixture was stirred, with smooth refluxing (90°C oil bath), for 2 hours.

After cooling down to rt, solid was filtered off *via* a celite layer and washed with toluene (75 ml).

The filtrate and washings were combined and partly concentrated *in vacuo* to get rid of most 2-propanol. Then it was washed with NaOH (1M, 15 ml) and brine, and dried.

With stirring at rt, a solution of oxalic acid (0.9 g, 10 mmol) in ethanol (1 ml) was added, the mixture was stirred at rt for 2 hours and further at ∼5°C overnight. Solid was collected by filtration and washed once with ethyl acetate (5 ml). 2.35 g oxalate solid was obtained after drying at 40°C overnight *in vacuo.*

### Example 4 (compound (4))

### Step 1 - compound (8a)

A mixture containing (4-chlorophenyl)phenyl methylamine hydrochloride salt (5.05 g, 20 mmol), bis(chloroethyl)amine phenyl carbamate (5.76 g, 22 mmol) and diisopropyl ethylamine (12 ml) was stirred at 80°C for 2 hours (N₂ gas was blown through during the reaction), and further at 100°C for 9 hours.

After cooling down to 50°C, 20 ml ethyl acetate was added. Mixture was stirred for 20 Min, while the temperature further went down to rt. Solid was filtered off and washed with Ethyl acetate (2x5 ml). Filtrate and washings were combined, and 50 ml ethyl acetate was added. The ethyl acetate solution was washed with H₂O (50 ml), NaOH (1M, 2x50 ml), H₂O (20 ml), brine (20 ml), dried and concentrated to give an oily material (8.8 g).

### Step 2 - compound (4)

Above crude material was dissolved in a solution containing NaOH (4 g) in 10 ml H₂O and 40 ml 2-propanol. With stirring, mixture was refluxed for 3hours.

The mixture was concentrated *in vacuo* to get rid of 2-propanol and redissolved in 100 ml ethyl acetate and 100 ml H₂O. The mixture was stirred for 20 min, and layers were separated. 20 ml HCl (2M) was added to the ethyl acetate layer and stirred for 20 min. Separated acid layer was basified. The mixture was extracted with ethyl acetate (2x50 ml). Combined ethyl acetate layer was washed with H₂O(10 ml), brine (10 ml), dried and concentrated *in vacuo* to give the desired product (2.5 g), which was solidified while seeded.

### Example 5 - compound (8a)

A mixture containing (4-chlorophenyl)phenyl methylamine hydrochloride (2.53 g, 10 mmol), bis(chloroethyl)amine phenyl carbamate (2.88 g, 11 mmol) and diisopropyl ethylamine (5 ml) was stirred (N₂ gas was pre-blown through for 1 hour before heating up), with smooth refluxing (130°C oil bath), for 6 hours.

After cooling down to room temperature, 50 ml ethyl acetate and 25 ml water was added. Mixture was stirred for 10 min and solid was filtered off. The filtrate was washed with HCl (1M, 15 ml), H₂O (15 ml), brine (15 ml), dried and concentrated to give a crude product (4.4 g).

The crude product was purified by chromatography (2% to 10% ethyl acetate in heptane were used as eluents). After removal of the solvent, the purified product solidified at rt (2.8 g). Analytical sample was obtained by washing of the solid with heptane.
¹H NMR (400 MHz, CDCl₃):
δ 7.40-7.05 (m, 14H, Ph-H), 4.27 (s, 1H, CH-piperazine),
3.7-3.5 (br, 4H, piperazine-H), 2.43 (m, 4H, piperazine-H).
EI/MS: 407 (M⁺+1), 201 (M⁺-N(CH₂CH₂)₂COOPh).

### Example 6 - compound (4) - R enatiomer

### Step 1 - Compound (8a) - R enantiomer

A mixture containing (R)- (4-chlorophenyl)phenyl methylamine free base (4.34 g, 20 mmol, optical purity 99%), bis(chloroethyl)amine phenyl carbamate (5.76 g, 22 mmol) and diisopropyl ethylamine (10 ml) was stirred under nitrogen at 90°C for 1 hour and further at 115°C for 5 hours.

After cooling down, 20 ml ethyl acetate was added. Mixture was stirred for 20 min, solid was filtered off and washed with ethyl acetate (2x25 ml). Filtrate and washings were combined, washed with H₂O (2x25 ml), and concentrated to give an oily material (8.9 g).

### Step 2 - Compound (4) - R enantiomer

Above crude material was dissolved in a solution containing 4 g NaOH in 10 ml H₂O and 40 ml 2-propanol. With stirring, mixture was refluxed for 3hours.

The mixture was concentrated *in vacuo* to get rid of 2-propanol and redissolved in 50 ml ethyl acetate and 20 ml H₂O. The mixture was stirred for 20 min, and layers were separated. 20 ml HCl (2M) was added to the ethyl acetate layer and stirred for 20 min. Separated ethyl acetate layer was extratected again with 10 ml HCl (1M). Acidic layers were combined and basified. The mixture was extracted with ethyl acetate (2x50 ml). Combined ethyl acetate layer was washed with H₂O(10 ml), brine (10 ml), dried and concentrated *in vacuo* to give the desired product (2.7 g), which was solidified while seeded. Optical purity ∼99%.

## Claims

1. A process, which comprises:
a) reacting a compound of formula (1) with a compound of formula (7) in the presence of a base, 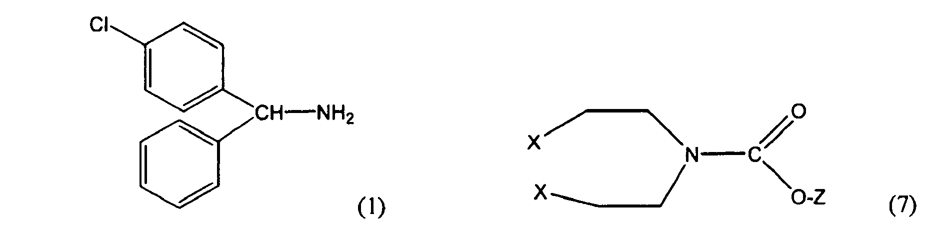 to form a compound of formula (8): 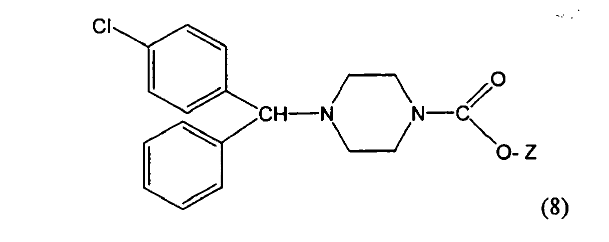 wherein X is a leaving group reactive with an amine, preferably a halo group such as chloro or bromo group; or a sulphonyl group such as mesyloxy, besyloxy or tosyloxy group; Z is a phenyl group; and
b) deprotecting said compound of formula (8) to form a compound of formula (4)

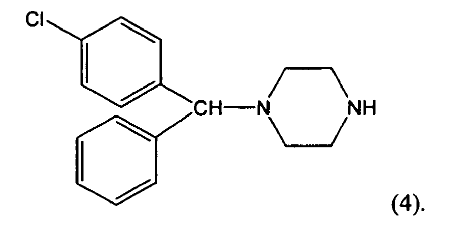

2. The process according to claim 1 wherein X is a chloro- group

3. The process according to claim 1 or 2, wherein the compounds of formulae (1), (8), and/or (4) are racemic compounds.

4. The process according to claim 1-3, which further comprises converting said compound of formula (4) to a cetirizine compound.

5. The process according to claim 1-4, wherein the compounds of formulae (1), (8), and/or (4) are substantially the (R)-enantiomer compounds.

6. The process according to claim 5, wherein said compound of formula (4) has an optical purity of at least 95%.

7. The process according to claim 1-6, which further comprises converting said compound of formula (4) to a levocetirizine compound.

8. The process according to claim 1-7, which further comprises isolating said compound of formula (4) as an oxalate salt, preferably in crystalline form.

9. The process according to claim 1-8 wherein the deprotection is performed without using HBr in acetic acid.

10. The process according to claim 1-9 wherein the deprotection is performed by an aqueous alkali.

11. A compound of formula (7) 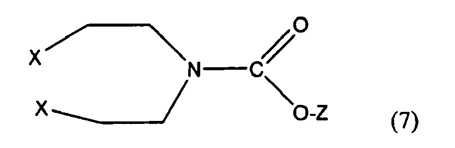 wherein X is a leaving group reactive with an amine, preferably a halo group such as chloro or bromo group; a sulphonyl group such as mesyloxy, besyloxy or tosyloxy group; and Z is a phenyl group.

12. The compound of claim 11, wherein X is a halo group selected from chloro or bromo group, or a sulphonyl group selected from mesyloxy, besyloxy or tosyloxy group.

13. The compound according to claim 11 or 12, wherein X is chloro.

14. A compound of formula (8) 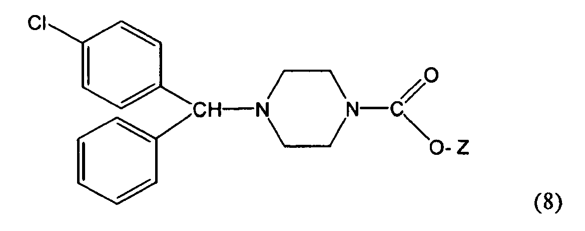 wherein Z is a phenyl group.

15. The compound according to claim 14, wherein said compound is substantially the (R) enantiomer. ,

16. The compound according to claim 14 or 15, having an optical purity of at least 95%.

17. A crystalline oxalic acid addition salt of a compound of formula (4)

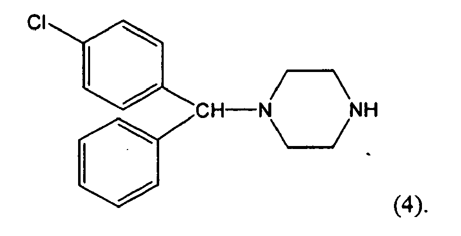

18. The crystalline salt according to claim 17, having an optical purity of at least 95%.

19. The crystalline salt of claim 17 or 18, which is the (R) enantiomer.

20. Use of a compound of claim 11, 14, 17, 18 or 19, in the preparation of cetirizide, and a pharmaceutically acceptable salt thereof.

## Patentansprüche

1. Verfahren, welches umfasst:
a) Umsetzen einer Verbindung der Formel (1) mit einer Verbindung der Formel (7) in Anwesenheit einer Base 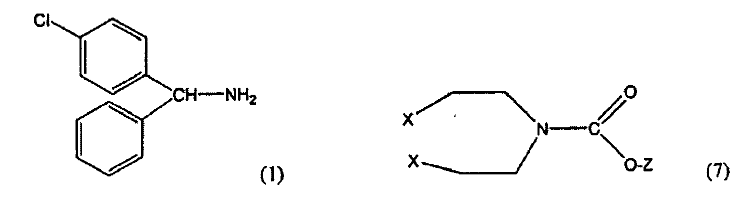 um eine Verbindung der Formel (8) zu bilden: 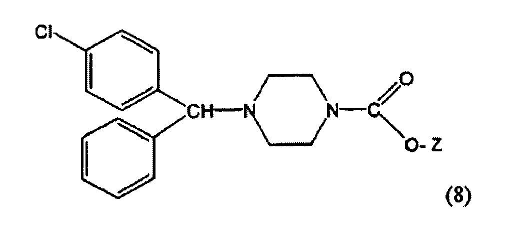 worin X eine Abgangsgruppe ist, die mit einem Amin reagiert, vorzugsweise eine Halogengruppe, wie eine Chlor- oder Bromgruppe; oder eine Sulfonylgruppe, wie eine Mesyloxy-, Besyloxy- oder Tosyloxygruppe; Z eine Phenylgruppe ist; und
b) Entschützen der Verbindung (8), um eine Verbindung der Formel (4) zu bilden

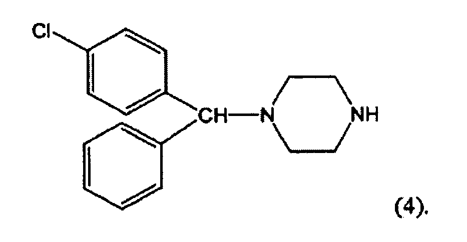

2. Verfahren nach Anspruch 1, bei dem X eine Chlorgruppe ist.

3. Verfahren nach Anspruch 1 oder 2, bei dem die Verbindungen der Formel (1), (8) und/oder (4) racemische Verbindungen sind.

4. Verfahren nach Anspruch 1-3, welches weiter das Umwandeln der Verbindung der Formel (4) in eine Cetirizin-Verbindung umfasst.

5. Verfahren nach Anspruch 1-4, bei dem die Verbindungen der Formel (1), (8) und/oder (4) im Wesentlichen die (R)-Enantiomer-Verbindungen sind.

6. Verfahren nach Anspruch 5, bei dem die Verbindung der Formel (4) eine optische Reinheit von mindestens 95 % besitzt.

7. Verfahren nach Anspruch 1-6, welches weiter die Umwandlung der Verbindung der Formel (4) in eine Levocetirizin-Verbindung umfasst.

8. Verfahren nach Anspruch 1-7, welches weiter die Isolierung der Verbindung der Formel (4) als Oxalatsalz, bevorzugt in kristalliner Form, umfasst.

9. Verfahren nach Anspruch 1-8, bei dem die Entschützung ohne Verwendung von HBr in Essigsäure durchgeführt wird.

10. Verfahren nach Anspruch 1-9, bei dem die Entschützung mittels eines wässrigen Alkalis durchgeführt wird

11. Verbindung der Formel (7) 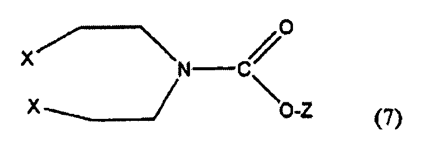 in der X eine Abgangsgruppe ist, die mit einem Amin reagiert, vorzugsweise eine Halogengruppe, wie eine Chlor- oder Bromgruppe, eine Sulfonylgruppe, wie eine Mesyloxy-, Besyloxy- oder Tosyloxygruppe, und Z eine Phenylgruppe ist.

12. Verbindung nach Anspruch 11, in der X eine Halogengruppe, die aus einer Chlor- oder Bromgruppe ausgewählt ist, oder eine Sulfonylgruppe ist, die aus einer Mesyloxy-, Besyloxy- oder Tosyloxygruppe ausgewählt ist.

13. Verbindung nach Anspruch 11 oder 12, in der X Chlor ist.

14. Verbindung der Formel (8) 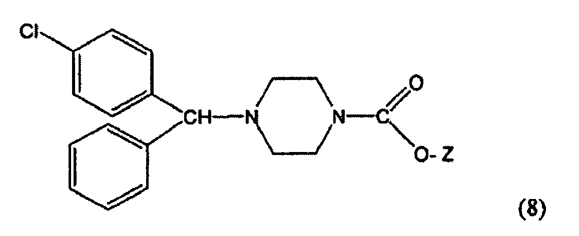 in der Z eine Phenylgruppe ist.

15. Verbindung nach Anspruch 14, in der die Verbindung im Wesentlichen das (R)-Enantiomer ist.

16. Verbindung nach Anspruch 14 oder 15 mit einer optischen Reinheit von mindestens 95 %.

17. Kristallines Oxalsäure-Additionssalz einer Verbindung der Formel (4)

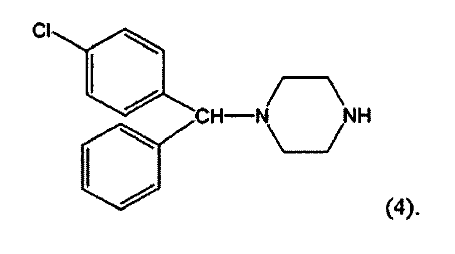

18. Kristallines Salz nach Anspruch 17 mit einer optischen Reinheit von mindestens 95 %.

19. Kristallines Salz nach Anspruch 17 oder 18, welches das (R)-Enantiomer ist.

20. Verwendung einer Verbindung nach Anspruch 11, 14, 17, 18 oder 19 bei der Herstellung von Cetirizid und einem pharmazeutisch annehmbaren Salz desselben.

## Revendications

1. Procédé qui comprend :
a) la mise en réaction d'un composé de formule (1) avec un composé de formule (7) en présence d'une base 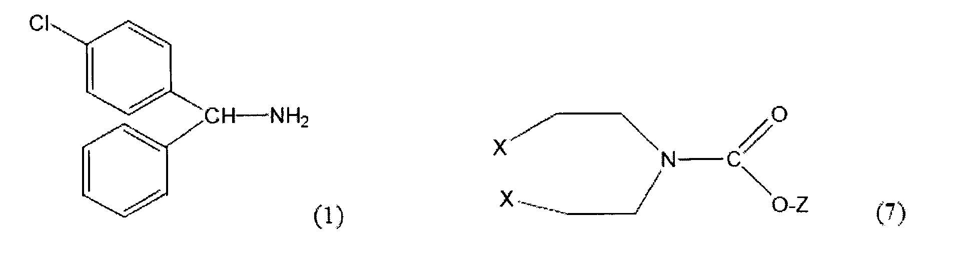 pour former un composé de formule (8) : 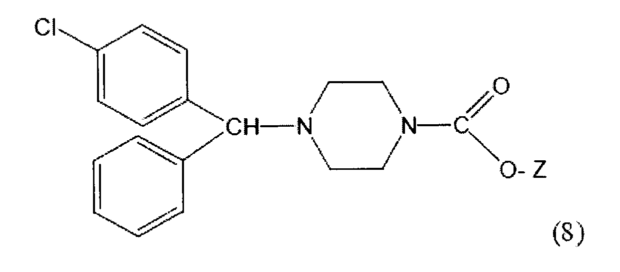 dans lesquelles X est un groupe partant réactif avec une amine, de préférence un groupe halogéno, tel qu'un groupe chloro ou bromo ; ou un groupe sulfonyle tel qu'un groupe mésyloxy, bésyloxy ou tosyloxy ; Z est un groupe phényle ; et
b) la déprotection dudit composé de la formule (8) pour former un composé de formule (4) :

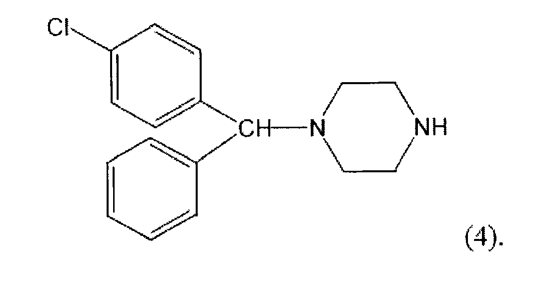

2. Procédé selon la revendication 1, dans lequel X est un group de chloro.

3. Procédé selon la revendication 1 ou 2, dans lequel les composés de formules (1), (8) et/ou (4) sont des composés racémiques.

4. Procédé selon l'une quelconque des revendications 1 à 3, qui comprend en outre la conversion dudit composé de formule (4) en un composé cétirizine.

5. Procédé selon l'une quelconque des revendications 1 à 4, dans lequel les composés de formules (1), (8) et/ou (4) sont essentiellement des composés énantiomères (R).

6. Procédé selon la revendication 5, dans lequel ledit composé de formule (4) possède une pureté optique d'au moins 95 %.

7. Procédé selon l'une quelconque des revendications 1 à 6, qui comprend en outre la conversion dudit composé de formule (4) en un composé lévocétirizine.

8. Procédé selon l'une quelconque des revendications 1 à 7, qui comprend en outre l'isolement dudit composé de formule (4) sous la forme d'un sel d'oxalate, de préférence sous une forme cristalline.

9. Procédé selon l'une quelconque des revendications 1 à 8, dans lequel la déprotection est réalisée sans utiliser d'HBr dans de l'acide acétique.

10. Procédé selon l'une quelconque des revendications 1 à 9, dans lequel la déprotection est réalisée par l'intermédiaire d'un alcali aqueux.

11. Composé de formule (7) : 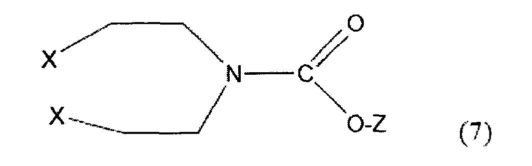 dans laquelle X est un groupe partant réactif avec une amine, de préférence un groupe halogéno, tel qu'un groupe chloro ou bromo ; un groupe sulfonyle tel qu'un groupe mésyloxy, bésyloxy ou tosyloxy ; et Z est un groupe phényle.

12. Composé selon la revendication 11, dans lequel X est un groupe halogéno choisi parmi un groupe chloro ou bromo, ou un groupe sulfonyle choisi parmi un groupe mésyloxy, bésyloxy ou tosyloxy.

13. Composé selon la revendication 11 ou 12, dans lequel X est un groupe chloro.

14. Composé de formule (8) : 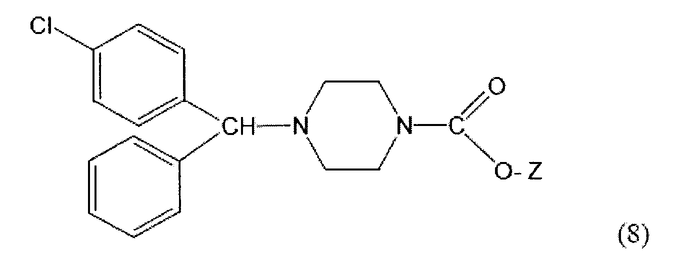 dans laquelle Z est un groupe phényle.

15. Composé selon la revendication 14, ledit composé étant essentiellement l'énantiomère (R).

16. Composé selon la revendication 14 ou 15, possédant une pureté optique d'au moins 95 %.

17. Sel d'addition d'acide oxalique cristallin d'un composé de formule (4) :

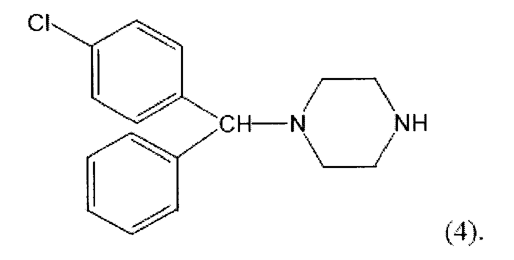

18. Sel cristallin selon la revendication 17, possédant une pureté optique d'au moins 95 %.

19. Sel cristallin selon la revendication 17 ou 18, qui est l'énantiomère (R).

20. Utilisation d'un composé selon la revendication 11, 14, 17, 18 ou 19, dans la préparation de cétirizide, et d'un sel pharmaceutiquement acceptable de celui-ci.
